(19) Europäisches Patentamt / European Patent Office / Office européen des brevets

(11) **EP 3 585 514 B1**

(12) **EUROPEAN PATENT SPECIFICATION**

(45) Date of publication and mention
of the grant of the patent:
**26.11.2025 Bulletin 2025/48**

(51) International Patent Classification (IPC):
**B01J 20/286** *(2006.01)*    **B01D 15/38** *(2006.01)*
**B01J 20/32** *(2006.01)*    **B01J 20/28** *(2006.01)*
**B01D 15/18** *(2006.01)*    **C07K 1/22** *(2006.01)*

(21) Application number: **18706693.1**

(22) Date of filing: **15.02.2018**

(52) Cooperative Patent Classification (CPC):
**B01J 20/286; B01D 15/3809; B01J 20/28004;**
**B01J 20/28011; B01J 20/28019; B01J 20/3274;**
**C07K 1/22;** B01D 15/1807; B01D 15/1821;
B01J 2220/52; B01J 2220/606

(86) International application number:
**PCT/EP2018/053816**

(87) International publication number:
**WO 2018/153772 (30.08.2018 Gazette 2018/35)**

(54) **A METHOD OF SEPARATING ANTIBODIES**

VERFAHREN ZUR TRENNUNG VON ANTIKÖRPERN

PROCÉDÉ DE SÉPARATION D'ANTICORPS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.02.2017 GB 201703116**

(43) Date of publication of application:
**01.01.2020 Bulletin 2020/01**

(73) Proprietor: **Cytiva BioProcess R&D AB**
**751 84 Uppsala (SE)**

(72) Inventors:
• **PALMGREN, Ronnie**
**751 84 Uppsala (SE)**
• **MALOISEL, Jean-Luc**
**751 84 Uppsala (SE)**
• **RODRIGO, Gustav**
**751 84 Uppsala (SE)**
• **BJORKMAN, Tomas**
**751 84 Uppsala (SE)**

(74) Representative: **Démoulin, Eva Lotta et al**
**Cytiva Sweden AB**
**Björkgatan 30**
**751 84 Uppsala (SE)**

(56) References cited:
WO-A1-2013/081540    WO-A1-2016/079033
WO-A1-2016/096790    US-A1- 2006 134 805
US-A1- 2013 020 263    US-B1- 6 602 990
US-B2- 7 901 581

SEQUENCE LISTING

<110> GE Healthcare Bio-Sciences AB

<120> A SEPARATION MATRIX AND A METHOD OF SEPARATING ANTIBODIES

<130> 318632

<160> 10

<170> PatentIn version 3.5

<210> 1
<211> 51
<212> PRT
<213> Staphylococcus aureus

<400> 1

```
Ala Gln Gln Asn Ala Phe Tyr Gln Val Leu Asn Met Pro Asn Leu Asn
1               5                   10                  15

Ala Asp Gln Arg Asn Gly Phe Ile Gln Ser Leu Lys Asp Asp Pro Ser
            20                  25                  30

Gln Ser Ala Asn Val Leu Gly Glu Ala Gln Lys Leu Asn Asp Ser Gln
            35                  40                  45

Ala Pro Lys
            50
```

<210> 2
<211> 61
<212> PRT
<213> Staphylococcus aureus

<400> 2

```
Ala Asp Ala Gln Gln Asn Lys Phe Asn Lys Asp Gln Gln Ser Ala Phe
1               5                   10                  15

Tyr Glu Ile Leu Asn Met Pro Asn Leu Asn Glu Glu Gln Arg Asn Gly
            20                  25                  30

Phe Ile Gln Ser Leu Lys Asp Asp Pro Ser Gln Ser Thr Asn Val Leu
            35                  40                  45

Gly Glu Ala Lys Lys Leu Asn Glu Ser Gln Ala Pro Lys
            50                  55                  60
```

<210> 3
<211> 58
<212> PRT
<213> Staphylococcus aureus

<400> 3

```
Ala Asp Asn Asn Phe Asn Lys Glu Gln Gln Asn Ala Phe Tyr Glu Ile
1               5               10              15

Leu Asn Met Pro Asn Leu Asn Glu Glu Gln Arg Asn Gly Phe Ile Gln
            20              25              30

Ser Leu Lys Asp Asp Pro Ser Gln Ser Ala Asn Leu Leu Ala Glu Ala
            35              40              45

Lys Lys Leu Asn Glu Ser Gln Ala Pro Lys
        50              55
```

<210> 4
<211> 58
<212> PRT
<213> Staphylococcus aureus

<400> 4

```
Ala Asp Asn Lys Phe Asn Lys Glu Gln Gln Asn Ala Phe Tyr Glu Ile
1               5               10              15

Leu His Leu Pro Asn Leu Asn Glu Glu Gln Arg Asn Gly Phe Ile Gln
            20              25              30

Ser Leu Lys Asp Asp Pro Ser Gln Ser Ala Asn Leu Leu Ala Glu Ala
            35              40              45

Lys Lys Leu Asn Asp Ala Gln Ala Pro Lys
        50              55
```

<210> 5
<211> 58
<212> PRT
<213> Staphylococcus aureus

<400> 5

```
Ala Asp Asn Lys Phe Asn Lys Glu Gln Gln Asn Ala Phe Tyr Glu Ile
1               5               10              15

Leu His Leu Pro Asn Leu Thr Glu Glu Gln Arg Asn Gly Phe Ile Gln
            20              25              30

Ser Leu Lys Asp Asp Pro Ser Val Ser Lys Glu Ile Leu Ala Glu Ala
            35              40              45

Lys Lys Leu Asn Asp Ala Gln Ala Pro Lys
        50              55
```

<210> 6
<211> 58

<212> PRT
<213> Escherichia coli

<400> 6

```
        Val Asn Asn Lys Phe Asn Lys Glu Gln Gln Asn Ala Phe Tyr Glu Ile
        1               5                   10                  15

        Leu His Leu Pro Asn Leu Asn Glu Glu Gln Arg Asn Ala Phe Ile Gln
                        20                  25                  30

        Ser Leu Lys Asp Asp Pro Ser Gln Ser Ala Asn Leu Leu Ala Glu Ala
                    35                  40                  45

        Lys Lys Leu Asn Asp Ala Gln Ala Pro Lys
                50                  55
```

<210> 7
<211> 58
<212> PRT
<213> Escherichia coli

<400> 7

```
        Val Asp Ala Lys Phe Asp Lys Glu Gln Gln Asn Ala Phe Tyr Glu Ile
        1               5                   10                  15

        Leu His Leu Pro Asn Leu Thr Glu Glu Gln Arg Asn Ala Phe Ile Gln
                        20                  25                  30

        Ser Leu Lys Asp Asp Pro Ser Gln Ser Ala Asn Leu Leu Ala Glu Ala
                    35                  40                  45

        Lys Lys Leu Asn Asp Ala Gln Ala Pro Lys
                50                  55
```

<210> 8
<211> 52
<212> PRT
<213> Escherichia coli

<400> 8

```
        Lys Glu Gln Gln Asn Ala Phe Tyr Glu Ile Leu His Leu Pro Asn Leu
        1               5                   10                  15

        Thr Glu Glu Gln Arg Asn Ala Phe Ile Gln Ser Leu Lys Asp Asp Pro
                        20                  25                  30

        Ser Gln Ser Ala Asn Leu Leu Ala Glu Ala Lys Lys Leu Asn Asp Ala
```

```
                           35                40                45

              Gln Ala Pro Lys
                   50

<210> 9
<211> 52
<212> PRT
<213> Escherichia coli

<400> 9

      Lys Glu Gln Gln Asn Ala Phe Tyr Glu Ile Leu His Leu Pro Asn Leu
      1               5               10                  15


      Thr Glu Glu Gln Arg Asn Gly Phe Ile Gln Ser Leu Lys Asp Asp Pro
                  20              25                  30


      Ser Val Ser Lys Glu Ile Leu Ala Glu Ala Lys Lys Leu Asn Asp Ala
                  35              40                  45


      Gln Ala Pro Lys
                  50

<210> 10
<211> 52
<212> PRT
<213> Escherichia coli

<400> 10

      Lys Glu Ala Gln Glu Ala Phe Tyr Glu Ile Leu His Leu Pro Asn Leu
      1               5               10                  15


      Thr Glu Glu Gln Arg Asn Ala Phe Ile Gln Ser Leu Lys Asp Asp Pro
                  20              25                  30


      Ser Val Ser Lys Ala Ile Leu Ala Glu Ala Lys Lys Leu Asn Asp Ala
                  35              40                  45


      Gln Ala Pro Lys
                  50
```

**Description**

Technical field of the invention

The present invention relates to a method of separating antibodies on the matrix.

Background of the invention

In the manufacturing of therapeutic monoclonal antibodies (mAbs), affinity chromatography on matrices comprising coupled *Staphylococcus* Protein A (SpA) or variants of SpA is commonly used as a first separation step to remove most of the contaminants. As the demand for therapeutic mAbs is increasing there is a strong driving force for improving the efficiencies of the separation processes and several approaches are under evaluation.

Multicolumn continuous chromatography processes are available, where the feed is applied to a first column and is then diverted to one or more subsequent columns as the first columns approaches saturation and the first column is eluted and regenerated to be loaded again during elution and regeneration of the subsequent column(s). Such processes can be denoted periodic countercurrent chromatography (PCC) or simulated moving bed (SMB) and are of considerable interest for separation of therapeutic mAbs, see e.g. US7901581, US20130248451, US20130280788 and US7220356. WO2013/081540 discloses affinity chromatography matrices for separation of immunoglobulins. PCC/SMB processes can significantly increase the productivity, but it appears that the full potential cannot be reached with currently available separation matrices, which are designed for conventional batch chromatography.

Accordingly, there is a need for new separation matrices specifically designed for continuous chromatography processes and for processes using such matrices. There is also a general need for separation matrices providing high dynamic binding capacities at very short residence times, in particular when used in shallow beds with low hydraulic resistance.

Summary of the invention

One aspect of the invention is to provide an efficient method of separating antibodies. This is achieved with a method as defined in the claims. One advantage is that the method allows very short residence times with high binding capacity.

Further suitable embodiments of the invention are described in the dependent claims.

Drawings

Fig. 1 shows an alignment of Protein A Fc-binding domains.

Fig. 2 shows the dynamic IgG-capacity vs residence time for prototype 128A compared with the 50 $\mu$m reference 871.

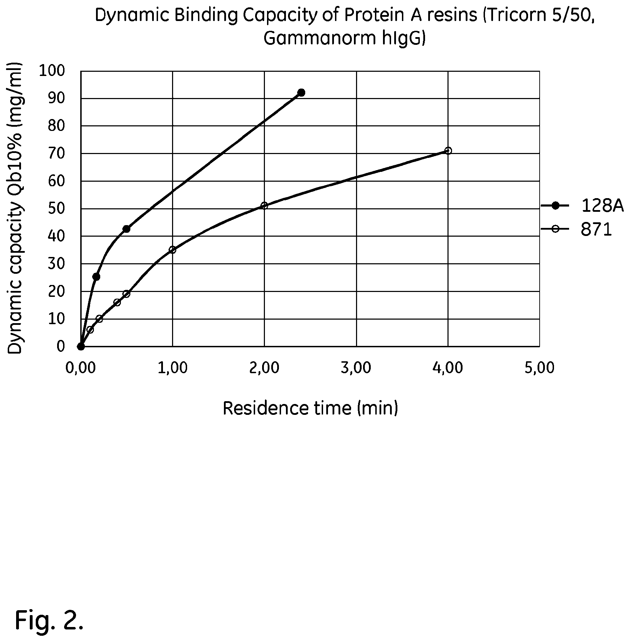

Fig. 2 shows the dynamic IgG-capacity vs residence time for prototypes 902B and 902A.

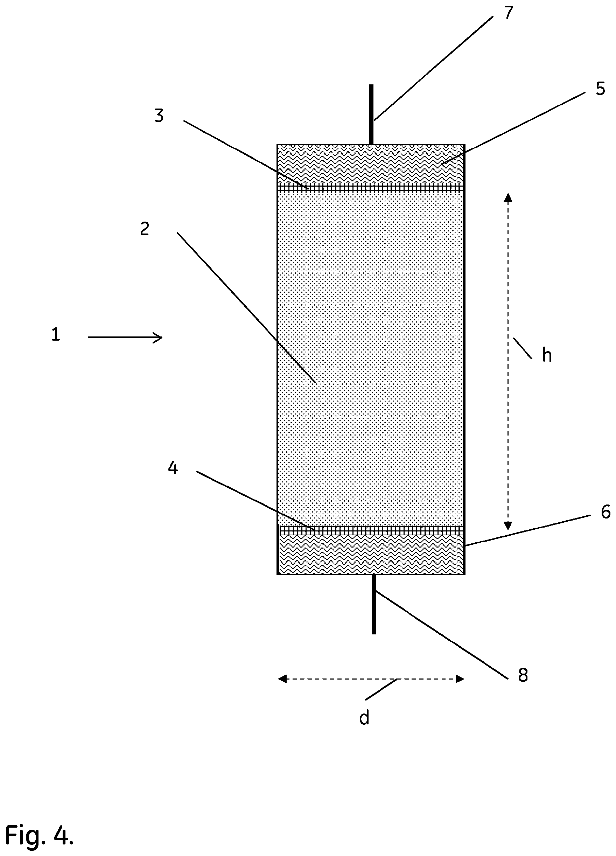

Fig. 4 shows a column according to the present disclosure.

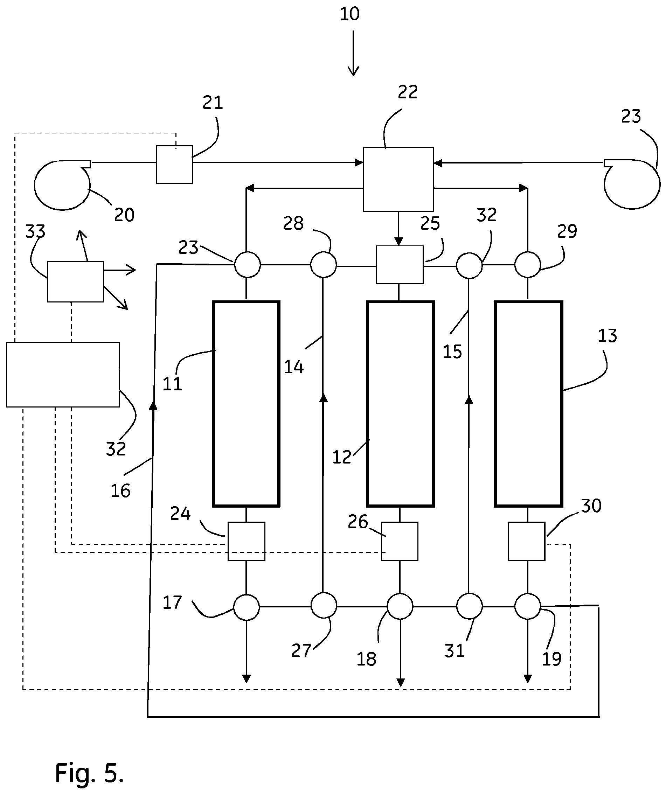

Fig. 5 shows a chromatography system according to the present disclosure.

Definitions

The terms "antibody" and "immunoglobulin" are used interchangeably herein, and are understood to include also fragments of antibodies, fusion proteins comprising antibodies or antibody fragments and conjugates comprising antibodies or antibody fragments.

The terms an "Fc-binding polypeptide" and "Fc-binding protein" mean a polypeptide or protein respectively, capable of binding to the crystallisable part (Fc) of an antibody and includes e.g. Protein A and Protein G, or any fragment or fusion protein thereof that has maintained said binding property.

The term "linker" herein means an element linking two polypeptide units, monomers or domains to each other in a multimer.

The term "spacer" herein means an element connecting a polypeptide or a polypeptide multimer to a support.

The term "% identity" with respect to comparisons of amino acid sequences is determined by standard alignment algorithms such as, for example, Basic Local Alignment Tool (BLAST™) described in Altshul et al. (1990) J. Mol. Biol., 215: 403-410. A web-based software for this is freely available from the US National Library of Medicine at http://blast.ncbi. nlmnih.gov/Blast.cgi?PROGRAM=blastp&PAGE_TYPE=BlastSearch&LINK _LOC=blasthome . Here, the algorithm "blastp (protein-protein BLAST)" is used for alignment of a query sequence with a subject sequence and determining i.a. the % identity.

As used herein, the terms "comprises," "comprising," "containing," "having" and the like can have the meaning ascribed to them in U.S. Patent law and can mean "includes," "including," and the like; "consisting essentially of or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

Detailed description of embodiments

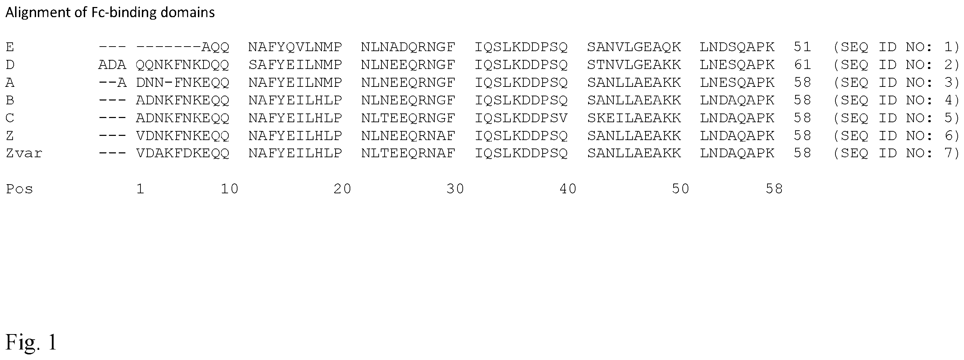

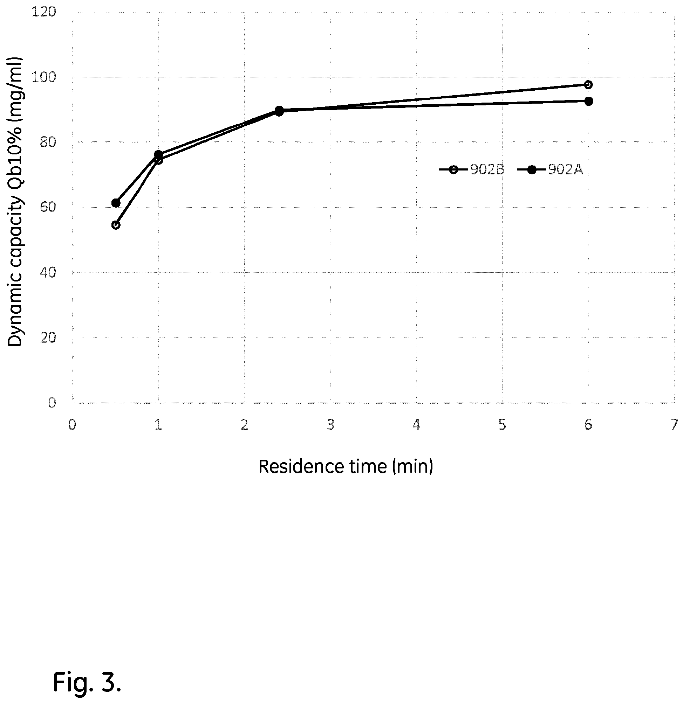

Figs. 1-3 disclose a separation matrix comprising porous, suitably spherical, particles to which antibody-binding protein ligands have been covalently immobilized. The density of these ligands is above 5 mg/ml, e.g. in the range of 5 - 25 mg/ml, such as 10.5-20 or 12-18 mg/ml, and the volume-weighted median diameter of the particles is at least 10 and below 30 $\mu$m, such as 10-29 $\mu$m or 15-28 $\mu$m. The density of the ligands denotes the content of coupled ligands per ml matrix sediment volume and is determined by amino acid analysis. The volume weighted median diameter, also denoted d50,v, is determined by electrozone sensing (Coulter Counter). A preferred method is to use electrozone sensing with an instrument calibrated with a narrow sieve fraction of the matrix in question, for which the d50,v, has been determined with microscopy and image analysis.

The porous particles may comprise a crosslinked polysaccharide, which provides a large hydrophilic surface for coupling of the ligands, with minimal risk of non-specific interactions between mAbs or contaminants and the particles. The polysaccharide suitably has zero or very low (e.g. < 5 micromol/ml) content of charged groups to prevent non-specific interactions. The crosslinking increases rigidity and chemical stability and can be achieved by methods known in the art, in particular by epoxide crosslinking, using e.g. epichlorohydrin or a diepoxide as crosslinker. Examples of polysaccharides can be dextran, cellulose and agarose. Agarose has the particular advantage that highly porous, rigid gels can be achieved by thermal gelation of aqueous agarose solution. The agarose can suitably be crosslinked by the methods described in US6602990, US7396467 or US8309709. Agarose crosslinked by these methods, so called high flow agarose, has a particularly advantageous combination of high rigidity and high porosity/pore volume, allowing high flow rates and rapid mass transport. High rigidity is particularly important for matrices having small particle sizes, to allow high flow rates without collapse of the matrix. The agarose can e.g. be allylated with reagents like allyl glycidyl ether or allyl halides before gelation, as described in US6602990. To allow for high binding capacities and rapid mass transport, the particles can advantageously have a large volume of pores accessible to macromolecular species like IgG antibodies. This can be determined by inverse size exclusion chromatography (SEC) as described in "Handbook of Process Chromatography, A Guide to Optimization, Scale-Up and Validation" (1997) Academic Press, San Diego, Gail Sofer & Lars Hagel eds. ISBN 0-12-654266-X, p. 368. A suitable parameter for the accessible pore volume is the gel phase distribution coefficient, $K_D$, determined for a probe molecule of defined size. This is a column-independent variable calculated from the retention volume $V_R$ for the probe molecule, the interstitial void volume of the column $V_0$ and the total liquid volume of the column Vt according to $K_D = (V_R-V_0)/(V_t-V_0)$. The porous particles have a $K_D$ value in the range of 0.6-0.85 such as 0.65-0.85, for dextran of molecular weight 110 kDa as the probe molecule.

The ligands can e.g. be derived from antibody-binding bacterial proteins or derivatives thereof, such as SpA (Protein A), *Peptostreptococcus* Protein L or *Streptococcus* Protein G.

Alternatively they can e.g. be derived from antibodies, such as single-chain camelid antibodies. They may bind to antibodies such that the affinity constant for the interaction is at most 1 x $10^{-6}$ M, for example at most 1 x $10^{-7}$ M, such as at most 1 x $10^{-9}$ M. They can comprise an Fc-binding protein, such as SpA or and SpA variant, which binds to the Fc part of IgG molecules. They can comprise monomers, dimers or multimers of native or mutated Protein A Fc-binding domains. The native Protein A Fc-binding domains E, D, A, B and C are shown in Fig. 1, together with the mutated variants Z and Zvar. In some embodiments, one or more of the domains in the ligands is derived from Protein Z or the B or C domain of Protein A, with the amino acid residue at position 23 being a threonine. Such domains have an improved alkali stability desirable for bioprocess use (see e.g. US8329860, US7834158, US 14/961164 and WO2016079033), which may e.g. be assessed by incubating the separation matrix 5 h in 0.5 M NaOH at 22 +/- 2 °C. Suitably, the matrix after incubation retains at least 90% or at least 95% of the original IgG-binding capacity. In certain embodiments, one or more of the domains comprises an amino acid sequence as defined by SEQ ID NO: 8, 9 or 10, or having at least 90% such as at least 95% or at least 98% identity to SEQ ID NO: 8, 9 or 10, as calculated by the BLAST algorithm. SEQ ID NO:8 is the Zvar domain minus the linker sequence VDAKFD and SEQ ID NO:9 is the C domain minus the linker sequence ADNKFN. SEQ ID NO:10 is a further mutation of SEQ ID NO:8. One or more of the domains, such as all the domains, may also be further mutated by one or more amino acid substitutions, insertions or deletions. Thus, for example, there may be up to 10, 9, 8, 7, 6, 5, 4, 3 or 2 mutations, e.g.

substitutions, within SEQ ID NO: 8, 9 or 10.

> SEQ ID NO:8
> KEQQ NAFYEILHLP NLTEEQRNAF IQSLKDDPSQ SANLLAEAKK LNDAQAPK
> SEQ ID NO:9
> KEQQ NAFYEILHLP NLTEEQRNGF IQSLKDDPSV SKEILAEAKK LNDAQAPK
> SEQ ID NO:10
> KEAQ EAFYEILHLP NLTEEQRNAF IQSLKDDPSV SKAILAEAKK LNDAQAPK

The ligands may comprise monomers, dimers or multimers of Protein A domains, typically wherein one or more of said domains have been mutated. One or more of the domains may e.g.be derived from Protein Z or the B or C domain of Protein A with the amino acid residue at position 23 being a threonine.

The ligands may additionally comprise one or more linker sequences of 1-10 amino acid residues, e.g.VDNKFN, ADNKFN, VDAKFD, AD or FN, suitably between the individual domains. In addition, the ligands may comprise a coupling moiety, e.g. a cysteine or a plurality of lysines at the C-terminus or N-terminus of the ligand, suitably at the C-terminus. The ligands may also comprise a leader sequence at the N-terminus, e.g. a scar or a residue after cleavage of a signal peptide and optionally also a copy of a linker sequence. Such a leader sequence may e.g. be a 1-15 amino acid (e.g. a 1-10 amino acid) peptide, e.g. AQ, AQGT, AQVDAKFD, AQGTVDAKFD or AQVDNKFN. Hence, a typical structure of a ligand may e.g. be Leader - (Domain-Linker)$_{n-1}$ - Domain - Coupling moiety. n may e.g. be 1-7, such as 1, 2, 3, 4, 5, 6 or 7.

As illustrated by Fig. 4, the present disclosure discloses a chromatography column 1 comprising the separation matrix as described above. The chromatography column can e.g. be an axial packed bed column **1,** where a cylindrical packed bed **2** of matrix particles is confined between two nets/frits **3,4** and two distributor structures **5,6,** allowing flow of a feed through an inlet **7,** an inlet distributor **5** and an inlet net/frit **3** through the packed bed **2** and then through an outlet frit/net **4,** an outlet distributor **6** and an outlet **8.** The height *h* of the packed bed is up to 5 cm, such as 2-5 cm, 2-4 cm or even 2-3 cm. The diameter *d* of the packed bed may e.g. be at least 0.5 or 1 cm, such as at least 1.5 cm or 1.5 - 200 cm, 1.5 - 100 cm, 1.5 - 50 cm or 1.5 - 30 cm. Suitably, the diameter/height ratio *d/h* may be >1, such as >2 or >3. This allows for reaching high capacities while keeping the back pressures low. The column can advantageously be a single use column, i.e. a pre-packed column constructed of low cost materials such as plastics, e.g. of polyolefins such as polypropylene and/or polyethylene. Scaling can be done by increasing the column diameter, thus increasing the *d/h* ratio as indicated above, but it is also possible to scale by using a plurality of columns coupled in parallel. Thus, the invention also discloses a plurality of columns as discussed above, coupled in parallel. Specific arrangements useful for parallel coupling of the columns are disclosed in e.g. US20120267299, US20130026100, US20130020263, US20133006867, US20140224738, US20140263012 and US 15/329199.

As illustrated by Fig. 5, the present disclosure also discloses a chromatography system **10** comprising a plurality of chromatography columns **11,12,13** as disclosed above (see also copending application PCT EP2016/069557). The system can suitably be arranged for performing continuous chromatography. It may e.g. comprise at least two, such as at least three chromatography columns **11,12,13** as disclosed above, packed with the same separation matrix and connected with one or more connecting lines **14,15,16** such that liquid can flow from one column **11,12** to a subsequent one **12,13** and from a last column **13** to a first column **11** and each connecting line between two columns may comprise at least one on/off valve **17,18,19,** which may be three-way or four-way valves. The system may further comprise a feed pump **20,** e.g. connected via a first detector **21** to a first valve block **22.** A buffer pump **23** may also be connected to this first valve block **22.** The first valve block **22** can further be connected to the inlet of a first column **11** via a first valve **23.** An outlet end of the first column **11** may be connected to a second valve **17** through a second detector **24.** The first valve block **22** can further be connected to the inlet of a second column **12** via a second valve or valve block **25.** An outlet end of the second column **12** can be connected to valve **18** via a third detector **26.** Furthermore, a valve **27** can be connected between valve **17** and valve **18.** Valve **27** can also be connected to a valve **28** which is also connected to valve **23** and the second valve block **25.** Hereby the effluent from the first column **11** can be directed to the inlet of the second column **12** through connecting line **14,** valves **17, 27, 28** and **25.** Furthermore the first valve block **22** can be connected to the inlet of a third column **13** via valve **29.** An outlet end of the third column **13** may be connected to valve **19** via a fourth detector **30.** Furthermore valve **31** can be connected between valve **18** and valve **19.** Valve **31** can also be connected to valve **32** which may also be connected to the second valve block **25** and valve **29.** Hereby the effluent from the second column **12** can be directed to the inlet of the third column **13** through connecting line **15.** The effluent from the third column **13** can be directed to the inlet of the first column **11** through connecting line **16** via valves **19** and **23** (alternatively it can be directed to a subsequent fourth column). Furthermore, the first, second, third and fourth detectors **21, 24, 26, 30** may all be connected to a determining unit **32.** The determining unit can be adapted to use the detected signals from the detectors to determine breakthrough and saturation points for the three different columns. The determining unit **32** and all the valve blocks, valves and pumps may further be connected to a control unit **33** (all the connections are not shown in the Figure) which is adapted to control the chromatography system in terms of when to remove or add columns from/into the loading zone, change flow rates, start new wash steps, etc. The detectors **21, 24, 26, 30** can e.g. be UV detectors. The control unit **33** may be configured to control the system according to breakthrough data obtained from the determining unit **32.** Alternatively, control unit **33** can use fixed predetermined step times for the switching operations.

The invention provides for a method of separation of antibodies by affinity chromatography according to claim 1.

The method can suitably be carried out in the chromatography system **10** disclosed above. It can typically be carried out as a capture step, following clarification of a cell culture supernatant feed comprising the antibodies. After step d), the recovered eluent with antibodies may be subjected to further chromatography steps, such as ion exchange, multimodal chromatography and/or hydrophobic interaction chromatography steps.

In certain embodiments of the method, in step a) an effluent from the first chromatography column **11** is passed through a second chromatography column **12** packed with the same separation matrix as the first column;

after step a), in a step a'), the process feed is redirected to the second chromatography column **12** and an effluent from the second chromatography column is passed through a third chromatography column **13** packed with the same separation matrix as the first and second columns;

after step a'), in a step a"), the process feed is redirected to the third chromatography column **13** and an effluent from the third chromatography column is passed through the first chromatography column **11;**

step c) is performed before step a");

after step a'), in a step c'), the eluent is conveyed through the second chromatography column **12** to elute antibodies;

after step a"), in a step c"), the eluent is conveyed through the third chromatography column **13** to elute antibodies; and

the sequence of steps a), a'), a"), c), c') and c") is optionally repeated one or more times.

The residence time in steps a), a') and a") is 0.3-1 min, such as 0.3-0.8 min.

The method may further, after steps c), c') and c") respectively, comprise steps e), e') and e"), each comprising conveying a cleaning liquid through said first, second and third chromatography columns respectively. The cleaning liquid can be an aqueous alkali solution comprising at least 0.1M (e.g. 0.1- 1M or 0.1 - 0.5 M) alkali. 0.5-1 M alkali solutions can also be used as sanitizing solutions. The alkali may e.g. be NaOH, but can also be e.g. KOH. The cleaning (also called cleaning in place - CIP) step ensures that any residual feed components are removed from the columns before repetition of the binding and elution steps. Suitably, the ligands are capable of withstanding repeated alkali treatments, e.g. as discussed above where the matrix retains at least 95% of its original IgG-binding capacity after 5 h incubation with 0.5 M NaOH.

After steps e), e') and e") respectively, the method may also comprise equilibration steps f), f') and f") to reequilibrate the columns for steps a), a') and a") respectively.

EXAMPLES

Example 1

Base matrices

The base matrices used were a set of rigid cross-linked agarose bead sieve fraction samples of 17-50 micrometers (volume-weighted, d50V) median diameter (determined on a Malvern Mastersizer 2000 laser diffraction instrument), prepared according to the methods of US6602990 and with a pore size corresponding to an inverse gel filtration chromatography Kd value of 0.50-0.82 for dextran of Mw 110 kDa, according to the methods described in "Handbook of Process Chromatography, A Guide to Optimization, Scale-Up and Validation" (1997) Academic Press, San Diego, Gail Sofer & Lars Hagel eds. ISBN 0-12-654266-X, p. 368., using HR10/30 columns (GE Healthcare) packed with the prototypes in 0.2 M NaCl and with a range of dextran fractions as probe molecules (flow rate 0.2 ml/min). The base matrix prototypes were sieved to obtain desired particle size distributions. Reference examples (Ref) are not according to the invention.

Table 1 Base matrix prototypes

| Prototype | Kd (dextran 110 kDa) | d50v ($\mu$m) |
|---|---|---|
| 989 (Ref) | 0.504 | 25 |
| 143 | 0.66 | 25 |
| 144 | | 25 |
| 743b | ~0.65 | 16.8 |

(continued)

| Prototype | Kd (dextran 110 kDa) | d50v ($\mu$m) |
|---|---|---|
| 743a | ~0.65 | 27.7 |
| 965 | 0.823 | 25 |
| P14 | 0.732 | 21.2 |
| 178 (Ref) | 0.65 | 50 |

Coupling

100 ml base matrix was washed with 10 gel volumes distilled water on a glass filter. The gel was weighed (1 g = 1 ml) and mixed with 30 ml distilled water and 8.08 g NaOH (0.202 mol) in a 250 ml flask with an agitator. The temperature was adjusted to 27 +/- 2 °C in a water bath. 16 ml epichlorohydrin (0.202 mol) was added under vigorous agitation (about 250 rpm) during 90 +/- 10 minutes. The reaction was allowed to continue for another 80 +/- 10 minutes and the gel was then washed with >10 gel volumes distilled water on a glass filter until neutral pH was reached. This activated gel was used directly for coupling as below.

To 16.4 mL of ligand solution (50 mg/mL) in a 50 ml Falcon tube, 139 mg $NaHCO_3$, 17.4 mg $Na_2CO_3$, 143.8 mg NaCl and 141 mg EDTA, was added. The Falcon tube was placed on a roller table for 5-10 min, and then 63 mg of DTE was added. Reduction proceeded for >45 min. The ligand solution was then desalted on a PD10 column packed with Sephadex G-25. The ligand content in the desalted solution was determined by measuring the 276 nm UV absorption.

The activated gel was washed with 3-5 GV {0.1 M phosphate/1 mM EDTA pH 8.6} and the ligand was then coupled according to the method described in US6399750 5.2.2, although with considerably higher ligand amounts (see below). All buffers used in the experiments had been degassed by nitrogen gas for at least 5-10 min. The ligand content of the gels was controlled by varying the amount and concentration of the ligand solution, adding 5-20 mg ligand per ml gel. The ligand was a tetramer of SEQ ID NO:8, with a VDAKFD linker and a C-terminal cysteine, except in prototypes 902A and B where it was a hexamer of SEQ ID NO:10 with a VDAKFD linker and a C-terminal cysteine.

After immobilization the gels were washed 3xGV (gel volumes) with distilled water. The gels + 1 GV {0.1 M phosphate/1 mM EDTA/10% thioglycerol pH 8.6} was mixed and the tubes were left in a shaking table at room temperature overnight. The gels were then washed alternately with 3xGV {0.1 M TRIS/0.15 M NaCl pH 8.6} and 0.5 M HAc and then 8-10xGV with distilled water. Gel samples were sent to an external laboratory for amino acid analysis and the ligand content (mg/ml gel) was calculated from the total amino acid content. Table 2 shows the ligand contents of the coupled prototypes and the Kd and d50v data for the corresponding base matrices.

Table 2 Coupled prototypes

| Prototype | Kd (dextran 110 kDa) | d50v ($\mu$m) | Ligand content (mg/ml) |
|---|---|---|---|
| 129A (Ref) | 0.504 | 25 | 7.1 |
| 129B (Ref) | 0.504 | 25 | 12.4 |
| 128A | 0.66 | 25 | 7.0 |
| 128B | 0.66 | 25 | 11.1 |
| 124A (Ref) | 0 | 25 | 6.9 |
| 124B (Ref) | 0 | 25 | 11.6 |
| 796A | ~0.65 | 16.8 | 7.5 |
| 796B | ~0.65 | 16.8 | 13.1 |
| 795A | ~0.65 | 27.7 | 7.1 |
| 795B | ~0.65 | 27.7 | 12.2 |
| 118 | 0.823 | 25 | 6.6 |
| 902A | 0.732 | 21.2 | 15.4 |
| 902B | 0.732 | 21.2 | ~20 |
| 871 (Ref) | 0.65 | 50 | 11 |

Evaluation

The Qb10 % dynamic capacity for polyclonal human IgG at 0.5, 1, 2.4 and 6 min residence time was determined as outlined below.

Protein

Gammanorm 165 mg/ml (Octapharma), diluted to 2mg/ml in Equilibration buffer.

Equilibration buffer

PBS Phosphate buffer 20 mM + 0.15 M NaCl, pH 7.4

Adsorption buffer

PBS Phosphate buffer 20 mM + 0.15 M NaCl, pH 7.4

Elution buffers

100 mM acetate pH 2.9

Dynamic binding capacity

0.5 or 1 ml of resin was packed in TRICORN™ 5/50 columns (bed height 2.5-5 cm). The breakthrough capacity was determined with an ÄKTAExplorer 10 system at residence times of 0.5-6 minutes, with flow rates adjusted according to the residence time desired for the resin volume in question. Equilibration buffer was run through the bypass column until a stable baseline was obtained. This was done prior to auto zeroing. Sample was applied to the column until a 100% UV signal was obtained. Then, equilibration buffer was applied again until a stable baseline was obtained.

Sample was loaded onto the column until a UV signal of 85% of maximum absorbance was reached. The column was then washed with 5 column volumes (CV) equilibration buffer at flow rate 0.5ml/min. The protein was eluted with 5 CV elution buffer at a flow rate of 0.5 ml/min. Then the column was cleaned with 0.5M NaOH at flow rate 0.2 ml/min and reequilibrated with equilibration buffer.

For calculation of breakthrough capacity at 10% ($q_{10\%}$), the equation below was used. $q_{10\%}$ is the amount of IgG that is loaded onto the column until the concentration of IgG in the column effluent is 10% of the IgG concentration in the feed.

$$q_{10\%} = \frac{C_0}{V_C}\left[V_{app} - V_{sys} - \int_{V_{sys}}^{V_{app}} \frac{A(V) - A_{sub}}{A_{100\%} - A_{sub}} * dv\right]$$

$A_{100\%}$ = 100% UV signal;
$A_{sub}$ = absorbance contribution from non-binding IgG subclass;
$A(V)$ = absorbance at a given applied volume;
$V_c$ = column volume;
$V_{app}$ = volume applied until 10% breakthrough;
$V_{sys}$ = system dead volume;
$C_0$ = feed concentration.

The dynamic binding capacity (DBC) at 10% breakthrough was calculated. The dynamic binding capacity (DBC) was calculated for 10 and 80% breakthrough.

Table 3 Dynamic capacity results

| Prototype | Bed height (mm) | Residence time (min) | Dynamic capacity, Qb10% (mg/ml) |
|-----------|-----------------|----------------------|--------------------------------|
| 129A (Ref) | 25 | 0.5 | 31.6 |
| 129B (Ref) | 27 | 0.5 | 21.2 |
| 128A | 25 | 0.5 | 42.6 |

(continued)

| Prototype | Bed height (mm) | Residence time (min) | Dynamic capacity, Qb10% (mg/ml) |
|---|---|---|---|
| 128A | 25 | 2.4 (Ref) | 92 |
| 128B | 26 | 0.5 | 47 |
| 128B | 26 | 2.4 (Ref) | 84.2 |
| 124A (Ref) | 24 | 0.5 | 41.4 |
| 124B (Ref) | 26 | 0.5 | 32.8 |
| 796A | 25 | 0.5 | 54 |
| 796A | 25 | 1.0 | 59 |
| 796A | 25 | 2.4 (Ref) | 62 |
| 796A | 25 | 6.0 (Ref) | 70 |
| 796B | 25 | 0.5 | 61 |
| 796B | 25 | 1.0 | 71 |
| 796B | 25 | 2.4 (Ref) | 81 |
| 796B | 25 | 6.0 (Ref) | 101 |
| 795A | 25 | 1.0 | 49 |
| 795A | 25 | 2.4 (Ref) | 55 |
| 795A | 25 | 6.0 (Ref) | 61 |
| 795B | 25 | 0.5 | 38 |
| 795B | 25 | 1.0 | 55 |
| 795B | 25 | 2.4 (Ref) | 67 |
| 795B | 25 | 6.0 (Ref) | 77 |
| 118 | 50 | 0.5 | 41 |
| 118 | 50 | 1.0 | 46 |
| 118 | 50 | 2.4 (Ref) | 52 |
| 902A | 50 | 0.5 | 54.7 |
| 902A | 50 | 1.0 | 74.6 |
| 902A | 50 | 2.4 (Ref) | 89.4 |
| 902A | 50 | 6.0 (Ref) | 97.7 |
| 902B | 50 | 0.5 | 61.4 |
| 902B | 50 | 1.0 | 76.2 |
| 902B | 50 | 2.4 (Ref) | 90 |
| 902B | 50 | 6.0 (Ref) | 92.7 |
| 871 (Ref) | 50 | 0.5 | 19 |
| 871 (Ref) | 50 | 1.0 | 35 |

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art.

## Claims

1.  A method of separation of antibodies by affinity chromatography, which method comprises the steps of:

    a) conveying a process feed through at least a first chromatography column comprising a packed bed of a separation matrix, wherein said packed bed has a bed height *h* of up to 5 cm, and wherein the separation matrix comprises porous particles to which antibody-binding protein ligands have been covalently immobilized, wherein the density of said ligands is above 5 mg/ml, wherein density of the ligands denotes the content of coupled ligands per ml matrix sediment volume as determined by amino acid analysis, the volume-weighted median diameter of said porous particles is at least 10 and below 30 $\mu$m as determined by electrozone sensing, and the said porous particles have a gel phase distribution coefficient, expressed as $K_D$ for dextran of molecular weight 110 kDa, of 0.6-0.85 as determined according to $K_D = (V_R-V_0)/(V_t-V_0)$, wherein $V_R$ is the retention volume for dextran, $V_0$ is interstitial void volume of the column, and $V_t$ is the total liquid volume of the column, to adsorb antibodies from said feed;

b) optionally washing said first chromatography column;

c) conveying an eluent through said first chromatography column to elute antibodies; and

d) recovering said eluent with antibodies,

wherein in step a) the residence time is 0.3-1 min .

2. The method of claim 1, wherein:

in step a) an effluent from said first chromatography column is passed through a second chromatography column packed with the same separation matrix as the first column;

after step a), in a step a'), the process feed is redirected to the second chromatography column and an effluent from the second chromatography column is passed through a third chromatography column packed with the same separation matrix as the first and second columns;

after step a'), in a step a"), the process feed is redirected to the third chromatography column and an effluent from the third chromatography column is passed through the first chromatography column;

step c) is performed before step a");

after step a'), in a step c'), the eluent is conveyed through the second chromatography column to elute antibodies;

after step a"), in a step c"), the eluent is conveyed through the third chromatography column to elute antibodies; and

the sequence of steps a), a'), a"), c), c') and c") is optionally repeated one or more times, wherein in steps a') and a"), the residence time is 0.3-1 min.

3. The method of any one of claims 1 or 2, wherein in step a), and optionally also in steps a') and a"), the residence time is 0.3-0.8 min.

4. The method of any one of claims 1-3, wherein said process feed comprises at least 4 mg/ml antibodies, such as 4-15 or 4-10 mg/ml.

5. The method of any one of claims 2-4, further comprising steps e), e') and e"), after steps c), c') and c") respectively, comprising conveying a cleaning liquid through said first, second and third chromatography columns, such as wherein said cleaning liquid comprises at least 0.1 M NaOH.

**Patentansprüche**

1. Verfahren zur Trennung von Antikörpern durch Affinitätschromatographie, wobei das Verfahren die Schritte umfasst zum:

a) Fördern eines Prozesszulaufs durch mindestens eine erste Chromatographiesäule, die ein Festbett einer Trennmatrix umfasst, wobei das Festbett eine Betthöhe **h** von bis zu 5 cm aufweist und wobei die Trennmatrix poröse Partikel umfasst, an die Antikörper-bindende Proteinliganden kovalent immobilisiert worden sind, wobei die Dichte der Liganden über 5 mg/ml beträgt, wobei Dichte der Liganden, wie durch Aminosäureanalyse bestimmt, den Gehalt an gekoppelten Liganden pro ml Matrixsedimentvolumen bezeichnet, der volumengewichtete mittlere Durchmesser der porösen Partikel, wie durch Elektrozonenmessung bestimmt, mindestens 10 und weniger als 30 $\mu$m beträgt, und die porösen Partikel einen Gelphasenverteilungskoeffizienten, ausgedrückt als $K_D$ für Dextran mit einem Molekulargewicht von 110 kDa, von 0,6-0,85 aufweisen, wie gemäß $K_D = (V_R-V_0)/(V_t-V_0)$ bestimmt, wobei $V_R$ das Retentionsvolumen für Dextran ist, $V_0$ das interstitielle Hohlraumvolumen der Säule ist und $V_t$ das gesamte Flüssigkeitsvolumen der Säule ist, um Antikörper aus dem Zulauf zu adsorbieren;

b) optionalen Waschen der ersten Chromatographiesäule;

c) Fördern eines Elutionsmittels durch die erste Chromatographiesäule, um Antikörper zu eluieren; und

d) Rückgewinnen des Elutionsmittels mit Antikörpern,

wobei in Schritt a) die Verweilzeit 0,3-1 min beträgt.

**2.** Verfahren nach Anspruch 1, wobei:

in Schritt a) ein Abfluss aus der ersten Chromatographiesäule durch eine zweite Chromatographiesäule geführt wird, die mit der gleichen Trennmatrix wie die erste Säule gepackt ist;
nach Schritt a) in einem Schritt a') der Prozesszulauf zur zweiten Chromatographiesäule umgeleitet wird und ein Abfluss aus der zweiten Chromatographiesäule durch eine dritte Chromatographiesäule geführt wird, die mit der gleichen Trennmatrix gepackt ist wie die erste und zweite Säule;
nach Schritt a') in einem Schritt a") der Prozesszulauf zur dritten Chromatographiesäule umgeleitet wird und ein Abfluss aus der dritten Chromatographiesäule durch die erste Chromatographiesäule geführt wird;
Schritt c) vor Schritt a") durchgeführt wird;
nach Schritt a') in einem Schritt c') das Elutionsmittel durch die zweite Chromatographiesäule gefördert wird, um Antikörper zu eluieren;
nach Schritt a") in einem Schritt c") das Elutionsmittel durch die dritte Chromatographiesäule gefördert wird, um Antikörper zu eluieren; und
die Abfolge der Schritte a), a'), a"), c), c') und c") optional einmal oder mehrmals wiederholt wird, wobei in den Schritten a') und a") die Verweilzeit 0,3-1 min beträgt.

**3.** Verfahren nach einem der Ansprüche 1 oder 2, wobei in Schritt a) und optional auch in den Schritten a') und a") die Verweilzeit 0,3-0,8 Minuten beträgt.

**4.** Verfahren nach einem der Ansprüche 1-3, wobei der Prozesszulauf mindestens 4 mg/ml Antikörper, wie beispielsweise 4-15 oder 4-10 mg/ml umfasst.

**5.** Verfahren nach einem der Ansprüche 2-4, das weiter jeweils die Schritte e), e') und e") nach den Schritten c), c') und c") umfasst, die Fördern einer Reinigungsflüssigkeit durch die erste, zweite und dritte Chromatographiesäule umfassen, wobei die Reinigungsflüssigkeit beispielsweise mindestens 0,1 M NaOH umfasst.

## Revendications

**1.** Procédé de séparation d'anticorps par chromatographie d'affinité, lequel procédé comprend les étapes consistant à :

a) transporter une charge de traitement à travers au moins une première colonne de chromatographie comprenant un lit garni d'une matrice de séparation, dans lequel ledit lit garni présente une hauteur de lit *h* allant jusqu'à 5 cm et dans lequel la matrice de séparation comprend des particules poreuses sur lesquelles des ligands protéiques de liaison aux anticorps ont été immobilisés de manière covalente, dans lequel la densité desdits ligands est supérieure à 5 mg/ml, dans lequel la densité des ligands désigne la teneur en ligands couplés par ml de volume de sédiment de matrice telle que déterminée par analyse des acides aminés, le diamètre médian pondéré en volume desdites particules poreuses est d'au moins 10 et de moins de 30 $\mu$m tel que déterminé par détection d'électrozone, et lesdites particules poreuses présentent un coefficient de distribution de phase gel, exprimé en $K_D$ pour le dextrane de poids moléculaire 110 kDa, de 0,6 à 0,85 tel que déterminé selon $K_D = (V_R-V_0)/(V_t-V_0)$, dans lequel $V_R$ est le volume de rétention du dextrane, $V_0$ est le volume de vide interstitiel de la colonne et $V_t$ est le volume de liquide total de la colonne pour adsorber des anticorps provenant de ladite charge ;
b) facultativement laver ladite première colonne de chromatographie ;
c) transporter un éluant à travers ladite première colonne de chromatographie pour éluer des anticorps ; et
d) récupérer ledit éluant avec des anticorps,

dans lequel à l'étape a) le temps de séjour est de 0,3 à 1 min.

**2.** Procédé selon la revendication 1, dans lequel :

à l'étape a) un effluent provenant de ladite première colonne de chromatographie est passé à travers une deuxième colonne de chromatographie remplie de la même matrice de séparation que la première colonne ;
après l'étape a), à une étape a'), la charge de traitement est redirigée vers la deuxième colonne de chromatographie et un effluent provenant de la deuxième colonne de chromatographie est passé à travers une troisième colonne de chromatographie remplie de la même matrice de séparation que les première et deuxième colonnes ;

après l'étape a'), à une étape a"), la charge de traitement est redirigée vers la troisième colonne de chromatographie et un effluent provenant de la troisième colonne de chromatographie est passé à travers la première colonne de chromatographie ;

l'étape c) est réalisée avant l'étape a") ;

après l'étape a'), à une étape c'), l'éluant est transporté à travers la deuxième colonne de chromatographie pour éluer des anticorps ;

après l'étape a"), à une étape c"), l'éluant est transporté à travers la troisième colonne de chromatographie pour éluer des anticorps ; et

la séquence des étapes a), a'), a"), c), c') et c") est facultativement répétée une ou plusieurs fois, dans lequel dans les étapes a') et a"), le temps de séjour est de 0,3 à 1 min.

3. Procédé selon l'une quelconque des revendications 1 ou 2, dans lequel à l'étape a) et, facultativement également aux étapes a') et a"), le temps de séjour est de 0,3 à 0,8 min.

4. Procédé selon l'une quelconque des revendications 1 à 3, dans lequel ladite charge de traitement comprend au moins 4 mg/ml d'anticorps, tels que 4-15 ou 4-10 mg/ml.

5. Procédé selon l'une quelconque des revendications 2 à 4, comprenant en outre les étapes e), e') et e"), après les étapes c), c') et c") respectivement, comprenant le transport d'un liquide de nettoyage à travers lesdites première, deuxième et troisième colonnes de chromatographie, tel que dans lequel ledit liquide de nettoyage comprend au moins 0,1 M de NaOH.

Alignment of Fc-binding domains

```
E        --- -------AQQ  NAFYQVLNMP  NLNADQRNGF  IQSLKDDPSQ  SANVLGEAQK  LNDSQAPK  51  (SEQ ID NO: 1)
D        ADA QQNKFNKDQQ  SAFYEILNMP  NLNEEQRNGF  IQSLKDDPSQ  STNVLGEAKK  LNESQAPK  61  (SEQ ID NO: 2)
A        --A DNN-FNKEQQ  NAFYEILNMP  NLNEEQRNGF  IQSLKDDPSQ  SANLLAEAKK  LNESQAPK  58  (SEQ ID NO: 3)
B        --- ADNKFNKEQQ  NAFYEILHLP  NLNEEQRNGF  IQSLKDDPSQ  SANLLAEAKK  LNDAQAPK  58  (SEQ ID NO: 4)
C        --- ADNKFNKEQQ  NAFYEILHLP  NLTEEQRNGF  IQSLKDDPSV  SKEILAEAKK  LNDAQAPK  58  (SEQ ID NO: 5)
Z        --- VDNKFNKEQQ  NAFYEILHLP  NLNEEQRNAF  IQSLKDDPSQ  SANLLAEAKK  LNDAQAPK  58  (SEQ ID NO: 6)
Zvar     --- VDAKFDKEQQ  NAFYEILHLP  NLTEEQRNAF  IQSLKDDPSQ  SANLLAEAKK  LNDAQAPK  58  (SEQ ID NO: 7)

Pos          1       10          20          30          40          50          58
```

Fig. 1

Dynamic Binding Capacity of Protein A resins (Tricorn 5/50, Gammanorm hIgG)

Fig. 2.

Fig. 3.

Fig. 4.

Fig. 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7901581 B
- US 20130248451 A
- US 20130280788 A
- US 7220356 B
- WO 2013081540 A
- US 6602990 B
- US 7396467 B
- US 8309709 B
- US 8329860 B
- US 7834158 B
- US 14961164 B
- WO 2016079033 A
- US 20120267299 A
- US 20130026100 A
- US 20130020263 A
- US 20133006867 A
- US 20140224738 A
- US 20140263012 A
- US 15329199 B
- EP 2016069557 W
- US 6399750522 A

**Non-patent literature cited in the description**

- **ALTSHUL et al.** *J. Mol. Biol.*, 1990, vol. 215, 403-410
- A Guide to Optimization, Scale-Up and Validation. Handbook of Process Chromatography. Academic Press, 1997, 368